# EUROPEAN PATENT APPLICATION

(11) **EP 3 854 476 A1**
(43) Date of publication of application: **28.07.2021**
(21) Application number: 19863199.6
(22) Date of filing: 24.09.2019
(51) Int. Cl.: B01J 23/06, C07B 61/00, C07C 1/20, C07C 11/167

(54) **CATALYST, AND METHOD FOR PRODUCING 1,3-BUTADIENE USING SAME**

(30) Priority: 21.09.2018 JP 2018177276
(71) Applicant: SEKISUI CHEMICAL CO., LTD., Osaka-shi Osaka 530-8565 (JP)
(72) Inventor: NISHIYAMA, Haruka, Tsukuba-shi, Ibaraki 300-4292 (JP); YAGIHASHI, Noritoshi, Tsukuba-shi, Ibaraki 300-4292 (JP); SHINMEI, Kenichi, Tsukuba-shi, Ibaraki 300-4292 (JP); DASANAYAKE ALUTHGE Rasika, Tsukuba-shi, Ibaraki 300-4292 (JP)
(74) Representative: Ter Meer Steinmeister & Partner
(86) International application number: PCT/JP2019/037282
(87) International publication number: WO 2020/059889

(57) **Abstract**

The present invention provides a catalyst comprising at least one first metal selected from the group consisting of Groups 3 to 6 of the periodic table, wherein an amount of Bronsted acid sites of the catalyst is 1.8 µmol/g or less.

## Description

### Technical Field

The present invention relates to a catalyst, and a method for producing 1,3-butadiene using the same.

### Background Art

1,3-Butadiene is used as a raw material of styrene-butadiene rubber (SBR) or the like. In conventional techniques, 1,3-butadiene is purified from a C4 fraction generated as a by-product in naphtha cracking for synthesizing ethylene from petroleum. However, the usage of petroleum is reduced in accordance with increase of the usage of shale gas, and as a result, the production of 1,3-butadiene obtained by the cracking is also reduced, and there is a demand for a method for producing 1,3-butadiene to be employed instead of the cracking of petroleum.

As such a method for producing 1,3-butadiene, for example, PTL 1 discloses a method for producing butadiene including generating butadiene from ethanol or a mixture of ethanol and acetaldehyde in the presence of a catalyst. The catalyst is a solid catalyst in which a component (A): a metal selected from the group consisting of Group 11 and Group 10, a component (B): a metal selected from the group consisting of Group 12 and lanthanide of Group 3, and a component (C): a metal selected from the group consisting of Group 4 are supported on a support. This literature describes an invention relating to a method for producing butadiene in which a content of the component (B) is 3 to 33 parts by mass and a content of the component (C) is 5 to 33 parts by mass with respect to 1 part by mass of the component (A).

PTL 1 describes that process in which biomass (such as corn or sugarcane) used as a petroleum-alternative raw material is fermented to produce butadiene through butanediol, butanol, butene or ethanol has been studied. PTL 1 also describes that the invention can reduce selectivity of an un-reusable by-product, and can increase a conversion rate of a raw material.

PTL 1 describes, regarding the catalyst, that the component (A) is preferably copper and nickel, the component (B) is preferably zinc and lanthanum, and the component (C) is preferably zirconium. In examples, it is described that a solid catalyst in which Cu, Zn and Zr are supported on silica, a solid catalyst in which Cu, La and Zr are supported on silica, and a solid catalyst in which Ni, Zn and Zr are supported on silica are used as the catalyst.

Besides, as for ethanol used as a raw material, it is described that, to N₂ gas flowing at a flow rate of 10 to 15 ml/min, ethanol is added in a ratio of 0.05 ml/min, or ethanol and acetaldehyde are added both in a ratio of 0.1 ml/min.

### Citation List

### Patent Literature

PTL 1: JP 2016-23141A

### Summary of Invention

### Technical Problem

According to the invention described in PTL 1, selectivity of 1,3-butadiene or the like is high, and a raw material conversion rate is also high. In PTL 1, however, ethanol used as a raw material is diluted by N₂ gas to a low concentration, which is not industrially applicable.

In addition, if ethanol used as the raw material has a high concentration in the invention described in PTL 1, the selectivity of 1,3-butadiene can be lowered.

Therefore, an object of the present invention is to provide means capable of retaining a raw material conversion rate and selectivity of 1,3-butadiene synthesized to be high even when ethanol used as a raw material has a high concentration.

### Solution to Problem

The present inventors have made earnest studies to solve the above-described problem. As a result, it was found that the above-described problem can be solved by using a catalyst obtained by combining prescribed metals, and thus, the present invention was accomplished. Specifically, the present invention has the following aspects:
[1] A catalyst comprising at least one first metal selected from the group consisting of Groups 3 to 6 of the periodic table, wherein the catalyst has an amount of Bronsted acid sites of 1.8 µmol/g or less.
[2] A catalyst comprising: at least one first metal selected from the group consisting of Groups 3 to 6 of the periodic table; at least one second metal selected from the group consisting of Groups 12 to 14 of the periodic table; and an ethylene suppressant.
[3] The catalyst according to [2], wherein the ethylene suppressant is at least one third metal selected from the group consisting of Groups 1 and 2 of the periodic table.
[4] The catalyst according to any one of [1] to [3], wherein the first metal is hafnium.
[5] The catalyst according to [3] or [4], wherein the third metal comprises at least one selected from the group consisting of Li, Na, K, Rb, Cs, Mg, Ca, Sr, and Ba.
[6] The catalyst according to any one of [3] to [5], wherein a content rate of the third metal to a total number of moles of the first metal, the second metal and the third metal is 5 to 30% by mole.
[7] The catalyst according to any one of [1] to [6], for synthesizing 1,3-butadiene from ethanol.
[8] The catalyst according to any one of [3] to [7], comprising a support on which the first metal, the second metal and the third metal are supported.
[9] A method for producing 1,3-butadiene, comprising contacting a raw material gas comprising ethanol with the catalyst according to any one of [1] to [8].
[10] The method for producing 1,3-butadiene according to [9], wherein a content rate of the ethanol in the raw material gas is 10% by volume or more with respect to 100% by volume of the raw material gas.
[11] The method for producing 1,3-butadiene according to [9] or [10], wherein a reaction for the contacting is performed at 200 to 800°C.

### Advantageous Effects of Invention

A catalyst of the present invention is used, for example, in a method for producing 1,3-butadiene from ethanol, and thus, 1,3-butadiene can be produced from a raw material gas having a high ethanol concentration with a raw material conversion rate and selectivity of 1,3-butadiene to be synthesized retained high.

### Brief Description of Drawing

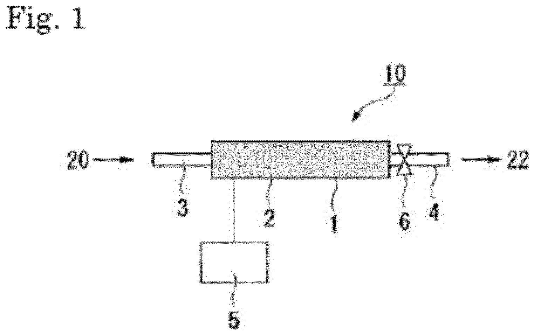

[Fig. 1] Fig. 1 is a schematic diagram of a production apparatus for 1,3-butadiene according to a method for producing 1,3-butadiene of the present invention.

### Description of Embodiment

In the appended claims and the present specification, the following definition of terms is applied.

The term "selectivity of 1,3-butadiene" refers to a percentage of the number of moles of a portion of a raw material converted into 1,3-butadiene in the number of moles of the raw material consumed in a reaction using a catalyst. When ethanol alone is used as the raw material, the number of moles of the raw material corresponds to the number of moles of ethanol. When ethanol and acetaldehyde are both used as the raw material, the number of moles of the raw material corresponds to a total number of moles of these.

The term "raw material conversion rate" refers to a percentage occupied by the number of consumed moles in the number of moles of a raw material contained in a raw material gas.

A numerical range indicated by using "to" embraces numerical values before and after "to" as a lower limit value and an upper limit value.

A catalyst of the present invention is used in a reaction for synthesizing 1,3-butadiene from a raw material gas containing, for example, ethanol. The catalyst may be in either of the following first and second aspects.

### [1. First Aspect]

A catalyst according to the first aspect of the present invention is a catalyst comprising at least one first metal selected from the group consisting of Groups 3 to 6 of the periodic table, and having an amount of Bronsted acid sites of 1.8 µmol/g or less.

When the amount of Bronsted acid sites exceeds 1.8 µmol/g, an amount of ethylene generated through dehydration of ethanol is increased in the reaction for synthesizing 1,3-butadiene from the raw material gas containing ethanol. As a result, selectivity of 1,3-butadiene is lowered. The amount of Bronsted acid sites can be adjusted by employing the second aspect described below.

The amount of Bronsted acid sites is preferably 1.7 µmol/g or less, and more preferably 1.6 µmol/g or less. The amount of Bronsted acid sites can be measured by a method described in an example below.

The catalyst according to the first aspect preferably comprises, particularly in a catalyst according to the second aspect described below, at least one first metal selected from the group consisting of Groups 3 to 6 of the periodic table, at least one second metal selected from the group consisting of Groups 12 to 14 of the periodic table, and at least one third metal selected from the group consisting of Groups 1 and 2 of the periodic table. For respective materials, a production method, and the like, the same conditions as those employed for the catalyst according to the second aspect can be employed.

### [2. Second Aspect]

The catalyst according to the second aspect of the present invention is a catalyst comprising at least one first metal selected from the group consisting of Groups 3 to 6 of the periodic table, at least one second metal selected from the group consisting of Groups 12 to 14 of the periodic table, and an ethylene suppressant.

Here, the ethylene suppressant refers to a component that suppresses generation of ethylene through dehydration of ethanol in the reaction for synthesizing 1,3-butadiene from the raw material gas containing ethanol. The ethylene suppressant is preferably at least one third metal selected from the group consisting of Groups 1 and 2 of the periodic table.

The catalyst according to the second aspect of the present invention preferably comprises at least one first metal selected from the group consisting of Groups 3 to 6 of the periodic table, at least one second metal selected from the group consisting of Groups 12 to 14 of the periodic table, and at least one third metal selected from the group consisting of Groups 1 and 2 of the periodic table.

Specific examples of the first metal include metals of Group 3 of the periodic table, such as Sc and Y, metals of Groups 4 of the periodic table, such as Ti, Zr, and Hf, metals of Group 5 of the periodic table, such as V, Nb, and Ta, and metals of Group 6 of the periodic table, such as Cr, Mo, and W. The first metal is preferably Zr, Hf and Ta, and more preferably Hf. As the first metal, one of these metals may be used singly, or two or more of these metals may be used in combination.

Specific examples of the second metal include metals of Group 12 of the periodic table, such as Zn, metals of Group 13 of the periodic table, such as B, Al, Ga, In, and Tl, and metals of Group 14 of the periodic table, such as Si, Ge, and Sn. The second metal is preferably Zn. As the second metal, one of these metals may be singly used, or two or more of these metals may be used in combination.

Specific examples of the third metal include metals of Group 1 of the periodic table, such as Li, Na, K, Rb, and Cs, and metals of Group 2 of the periodic table, such as Mg, Ca, Sr, and Ba. The third metal is preferably Li, Na, K, Rb, Cs, Mg, Ca, Sr, and Ba, more preferably Li, Na, K, and Cs, particularly preferably Li, Na, and Cs, and most preferably Na. As the third metal, one of these metals may be singly used, or two or more of these metals may be used in combination. When at least one metal selected from the group consisting of Groups 1 and 2 of the periodic table is used as the third metal, the raw material conversion rate and the selectivity of 1,3-butadiene can be increased in producing 1,3-butadiene from a raw material gas having a high ethanol concentration. When the third metal is Li, Na, or Cs, and preferably Na, the selectivity of 1,3-butadiene is further increased, and thus, a yield of 1,3-butadiene can be improved.

Specific examples of the catalyst include, but are not especially limited to, Zr-Zn-Li, Zr-Zn-Na, Zr-Zn-K, Zr-Zn-Cs, Hf-Zn-Li, Hf-Zn-Na, Hf-Zn-K, Hf-Zn-Cs, Ta-Zn-Li, Ta-Zn-Na, Ta-Zn-K, and Ta-Zn-Cs. Among these, Zr-Zn-Li, Zr-Zn-Na, Zr-Zn-Cs, Hf-Zn-Li, Hf-Zn-Na, Hf-Zn-Cs, Ta-Zn-Li, Ta-Zn-Na, and Ta-Zn-Cs are preferred, and Zr-Zn-Na, Hf-Zn-Na, and Ta-Zn-Na are more preferred. In another embodiment, the catalyst is preferably Hf-Zn-Li, Hf-Zn-Na, Hf-Zn-K, or Hf-Zn-Cs, more preferably Hf-Zn-Li, Hf-Zn-Na, or Hf-Zn-Cs, and further preferably Hf-Zn-Na.

A content rate of the first metal to the total number of moles of the first metal, the second metal and the third metal is not especially limited, and is preferably 40 to 90% by mole, more preferably 50 to 90% by mole, further preferably 50 to 85% by mole, particularly preferably 60 to 80% by mole, and most preferably 60 to 75% by mole.

When the content rate of the first metal falls in the above-described range, the raw material conversion rate and the selectivity of 1,3-butadiene can be increased in producing 1,3-butadiene from a raw material gas having a high ethanol concentration.

A content rate of the second metal to the total number of moles of the first metal, the second metal, and the third metal is not especially limited, and is preferably 5 to 30% by mole, more preferably 5 to 25% by mole, further preferably 7.5 to 25% by mole, and particularly preferably 10 to 20% by mole.

When the content rate of the second metal falls in the above-described range, the raw material conversion rate and the selectivity of 1,3-butadiene can be increased in producing 1,3-butadiene from a raw material gas having a high ethanol concentration.

A content rate of the third metal to the total number of moles of the first metal, the second metal, and the third metal is preferably 5 to 30% by mole, more preferably 5 to 25% by mole, further preferably 12 to 25% by mole, particularly preferably 12 to 20% by mole, and particularly preferably 12 to 15% by mole.

The content rate of the third metal preferably falls in the above-described range because thus, the selectivity of 1,3-butadiene can be increased and the yield of 1,3-butadiene can be improved.

It is noted that each of the "first metal", the "second metal", and the "third metal" herein may contain a metal in which at least a part thereof is an oxide. For example, assuming that the first metal is Hf, the second metal is Zn, and the third metal is Na (namely, in Hf-Zn-Na), when Zn used as the second metal is partially oxidized to be zinc oxide (ZnO), the second metal consists of Zn and ZnO. Besides, in Hf-Zn-Na, when the second metal is completely oxidized, the second metal consists of ZnO. A method for oxidizing at least a part of each of the "first metal", the "second metal", and the "third metal" is, for example, baking described below.

The catalyst of the present invention may comprise a metal or a metalloid in addition to the first to third metals as long as the effects of the present invention are not impaired.

The catalyst of the present invention may be an aggregate or a condensate of the catalytic metals, or may be a supported catalyst in which the catalytic metals (the first metal, the second metal, and the third metal) are supported on a support.

When the catalyst of the present invention is a supported catalyst, any of known supports can be used as the support of the metal catalyst. The support is a porous support such as silica, titania, and alumina. In particular, a preferable porous support is silica. Various silica products having different specific surface areas and pore diameters are commercially available. The selectivity of 1,3-butadiene and the raw material conversion rate can be controlled in accordance with a combination of a specific surface area and a pore diameter of the porous support.

The size of the porous support is not especially limited, and the particle size is preferably 0.1 to 1000 µm, and more preferably 0.1 to 100 µm. The particle size of the porous support is adjusted by sieving. The porous support preferably has a particle size distribution as narrow as possible. The particle size of the porous support is measured with a scanning electron microscope (SEM).

A sum of pore volumes (total pore volume) of the porous support is preferably 0.10 to 2.50 mL/g, and more preferably 0.25 to 1.50 mL/g. When the total pore volume is equal to or larger than the lower limit value, a sufficient amount of catalytic metals is easily supported, and the raw material conversion rate and the selectivity of 1,3-butadiene are further increased. When the total pore volume is equal to or smaller than the upper limit value, a sufficient contact area is easily obtained between the raw material and the metal catalyst, and the raw material conversion rate and the selectivity of 1,3-butadiene are further increased.

It is noted that the total pore volume of the porous support is a value measured by a water titration method. In the water titration method, water molecules are adsorbed onto the surface of the porous support, and a pore distribution is measured based on condensation of the molecules.

An average pore diameter of the porous support is preferably 0.1 to 100 nm, and more preferably 1.0 to 50 nm. When the average pore diameter is equal to or larger than the lower limit value, a sufficient amount of catalytic metals is easily supported, and the raw material conversion rate and the selectivity of 1,3-butadiene are further increased. When the average pore diameter is equal to or smaller than the upper limit value, a sufficient contact area is easily obtained between the raw material gas and the catalytic metals, and the raw material conversion rate and the selectivity of 1,3-butadiene are further increased.

It is noted that the average pore diameter is a value measured as follows: When the average pore diameter is 0.1 nm or more and less than 50 nm, the average pore diameter is calculated based on the total pore volume and a BET specific surface area. When the average pore diameter is 50 nm or more, the average pore diameter is measured with a porosimeter by a mercury press-in method. A BET specific surface area is a value calculated, by using nitrogen as an adsorbed gas, based on an adsorption amount of the gas and a pressure at the time of the adsorption. In the mercury press-in method, a pressure is applied to mercury to press the mercury into the pores of the porous support, and the average pore diameter is calculated based on the pressure and the amount of mercury pressed in.

The specific surface area of the porous support is preferably 50 to 1000 m²/g, and more preferably 100 to 750 m²/g. When the specific surface area is equal to or larger than the lower limit value, a sufficient amount of the catalytic metals is easily supported, and the raw material conversion rate and the selectivity of 1,3-butadiene are further increased. When the specific surface area is equal to or smaller than the upper limit value, a sufficient contact area is easily obtained between the raw material gas and the catalytic metals, and the raw material conversion rate and the selectivity of 1,3-butadiene are further increased.

It is noted that the specific surface area refers to a BET specific surface area measured by using nitrogen as the absorbed gas and employing the BET gas adsorption method.

A product of the total pore volume and the specific surface area of the porous support is preferably 5 to 7500 mL·m²/g², and more preferably 100 to 5000 mL·m²/g². When the product is equal to or larger than the lower limit value, a sufficient amount of the catalytic metals is easily supported, and the raw material conversion rate and the selectivity of 1,3-butadiene are further increased. When the product is equal to or smaller than the upper limit value, a sufficient contact area is easily obtained between the raw material gas and the catalytic metals, and the raw material conversion rate and the selectivity of 1,3-butadiene are further increased.

The catalyst of the present invention can be produced in accordance with any known method for producing a catalyst except that the first to third metals are used as the catalytic metals. When the catalyst of the present invention is obtained as a supported catalyst, for example, an impregnation method, a coprecipitation method, an ion exchange method or the like can be employed.

In the impregnation method, a porous support is impregnated with an impregnation solution containing the first and second metals, and the resultant porous support is dried and subjected to a baking treatment. Subsequently, the fired porous support is impregnated with an impregnation solution containing the third metal, and the resultant support is dried to obtain the catalyst of the present invention. After the support is impregnated with the third metal and dried, the resultant support may be further fired.

It is noted that an impregnation solution can be prepared, for example, by dissolving a raw material compound of each catalytic metal in a solvent.

The raw material compound of each catalytic metal is not especially limited, and any compound usually used for preparing a metal catalyst can be used. Specific examples of the raw material compound include an inorganic salt such as an oxide, a chloride, a sulfide, a nitrate, or a carbonate, an organic salt such as oxalate, acetylacetonate salt, dimethylglyoxime salt, or ethylenediamine acetate, or a chelate compound, a carbonyl compound, a cyclopentadienyl compound, an ammine complex, an alkoxide compound, or an alkyl compound.

Specific examples of the solvent include water, methanol, ethanol, tetrahydrofuran, dioxane, hexane, benzene, and toluene.

Specific examples of a method for impregnating the porous support with the impregnation solution include a simultaneous method and a consecutive method. The simultaneous method is a method in which a support is impregnated with a solution in which all raw material compounds are dissolved. The consecutive method is a method in which solutions respectively containing raw material compounds dissolved therein are prepared to consecutively impregnating the support with the respective solutions.

A drying temperature and a baking temperature can be appropriately determined in accordance with the type of the solvent or the like. For example, when the solvent is water, the drying temperature may be 80 to 120°C, and the baking temperature may be 300 to 600°C.

One of the above-described catalysts may be singly used, or two or more of these may be used in combination.

A production apparatus for 1,3-butadiene used in the production method of the present invention includes a reaction tube to be filled with the catalyst of the present invention. The production apparatus produces 1,3-butadiene from a raw material gas containing ethanol.

Now, an embodiment of the production apparatus will be described with reference to Fig. 1.

The production apparatus 10 for 1,3-butadiene (hereinafter simply referred to as the "production apparatus 10") of the present embodiment includes a reaction tube 1, a supply tube 3, a discharge tube 4, a temperature controller 5, and a pressure controller 6.

The reaction tube 1 includes a reaction bed 2 therein. The reaction bed 2 is filled with the catalyst of the present invention. The supply tube 3 is connected to the reaction tube 1. The discharge tube 4 is connected to the reaction tube 1. The temperature controller 5 is connected to the reaction tube 1. The discharge tube 4 includes the pressure controller 6.

The reaction bed 2 may include the catalyst of the present invention alone, or may include the catalyst of the present invention and a diluent. The diluent prevents excessive heat generation from the catalyst.

Here, a reaction for synthesizing 1,3-butadiene from a raw material gas containing ethanol is an endothermic reaction. Therefore, the reaction bed 2 usually does not need a diluent.

The diluent is, for example, one similar to that used as a support of a synthetic catalyst, or quartz sand, an alumina ball, an aluminum shot, or the like.

When the diluent is filled in the reaction bed 2, a mass ratio represented by "diluent/present catalyst" is determined in consideration of the types, specific gravities and the like of them, and is, for example, preferably 0.5 to 5.

It is noted that the reaction bed may be any one of a fixed bed, a moving bed, a fluidized bed and the like.

The reaction tube 1 is preferably made of a material inactive to the raw material gas and a synthesized product. The reaction tube 1 is preferably in a shape capable of withstanding heat of about 200 to 800°C, or a pressure of about 10 MPa. The reaction tube 1 is, for example, a substantially cylindrical member of stainless steel.

The supply tube 3 is supply means for supplying the raw material gas into the reaction tube 1. The supply tube 3 is, for example, a pipe of stainless steel or the like.

The discharge tube 4 is discharge means for discharging a gas containing the product synthesized in the reaction bed 2. The discharge tube 4 is, for example, a pipe of stainless steel or the like.

The temperature controller 5 may be any part as long as the reaction bed 2 of the reaction tube 1 can be set to an arbitrary temperature. The temperature controller 5 is, for example, an electric furnace or the like.

The pressure controller 6 may be any part as long as a pressure within the reaction tube 1 can be set to an arbitrary pressure. The pressure controller 6 is, for example, a known pressure valve or the like.

It is noted that the production apparatus 10 may include any known devices including a gas flow controller for adjusting a gas flow rate, such as a mass flow, or the like.

The method for producing 1,3-butadiene of the present invention is a method for producing 1,3-butadiene from a raw material by using the catalyst of the present invention described above.

In the method for producing 1,3-butadiene, the raw material gas containing ethanol is brought into contact with the catalyst of the present invention.

The raw material gas is a substance that can be converted into 1,3-butadiene, and contains at least ethanol. The raw material gas preferably contains, for example, ethanol, or ethanol and acetaldehyde.

A form in which the raw material gas is brought into contact with the catalyst of the present invention is not especially limited, and for example, the raw material gas is caused to pass through the reaction bed within the reaction tube to be brought into contact with the catalyst of the present invention in the reaction bed.

The raw material gas may contain a gas (an arbitrary gas) in addition to the raw material. An example of the arbitrary gas includes an inert gas such as nitrogen or argon.

The inert gas is a diluted gas. When the raw material gas contains a diluted gas, the selectivity of 1,3-butadiene is further increased.

A content rate of ethanol in the raw material gas is, with respect to 100% by volume of the raw material gas, preferably 10% by volume or more, more preferably 20% by volume or more, and further preferably 30% by volume or more. Although there is no especial upper limit in the content rate of ethanol in the raw material gas, the upper limit is preferably 90% by volume or less, more preferably 80% by volume or less, and further preferably 70% by volume or less. In the conventional method for producing ethanol using ethanol, a content rate of ethanol in the raw material gas is so low that the production method is difficult to be industrially applied. Besides, even if the content rate of ethanol is increased, a satisfactory raw material conversion rate and satisfactory selectivity of 1,3-butadiene cannot be simultaneously attained. When the above-described catalyst is used, however, even if the content rate of ethanol in the raw material gas is 10% by volume or more, the raw material conversion rate and the selectivity of 1,3-butadiene can be both increased.

When the raw material gas contains acetaldehyde, a content rate of acetaldehyde in the raw material gas is, with respect to 100% by volume of the raw material gas, preferably 0.1 to 50% by volume, and more preferably 0.5 to 30% by volume.

When the raw material gas contains an inert gas, a content rate of the inert gas is, with respect to 100% by volume of the raw material gas, preferably 30 to 90% by volume, more preferably 40 to 80% by volume, and particularly preferably 50 to 70% by volume. When the content of the inert gas is equal to or larger than the lower limit value, the selectivity of 1,3-butadiene is further increased. When the content of the inert gas is equal to or smaller than the upper limit value, a production amount per unit time of 1,3-butadiene is further increased.

A temperature at which the raw material gas is brought into contact with the catalyst of the present invention (a reaction temperature) is 200 to 800°C, preferably 375 to 800°C, more preferably 375 to 600°C, and particularly preferably 375 to 550°C. When the reaction temperature is equal to or higher than the lower limit value, a reaction speed is sufficiently increased, and 1,3-butadiene can be more efficiently produced. When the reaction temperature is equal to or lower than the upper limit value, deterioration of the catalyst of the present invention is easily suppressed. There is a tendency, in general, that as the reaction temperature is increased, the conversion rate is improved but the selectivity of 1,3-butadiene is lowered. When the above-described catalyst is used, however, as the reaction temperature is increased, the conversion rate is improved as well as the lowering of the selectivity of 1,3-butadiene can be suppressed or prevented, and as a result, 1,3-butadiene can be produced in a high yield.

A pressure at which the raw material gas is brought into contact with the catalyst of the present invention (a reaction pressure) is, for example, preferably 0.05 to 10 MPa, and more preferably 0.05 to 3 MPa. When the reaction pressure is equal to or higher than the lower limit value, the reaction speed is increased, and 1,3-butadiene can be more efficiently produced. When the reaction pressure is equal to or lower than the upper limit value, the deterioration of the catalyst of the present invention is easily suppressed.

A space velocity (SV) of the raw material gas in the reaction bed is, in terms of standard state, preferably 0.1 to 10000 L/h/catalyst volume (L-catalyst), more preferably 10 to 5000 L/h/catalyst volume (L-catalyst), and particularly preferably 100 to 2500 L/h/catalyst volume (L-catalyst). The space velocity is appropriately adjusted in consideration of the reaction pressure and the reaction temperature.

For example, when 1,3-butadiene is produced by using the production apparatus 10, the temperature controller 5 and the pressure controller 6 are used to set the inside of the reaction tube 1 to an arbitrary temperature and an arbitrary pressure. A raw material gas 20 in a gas form is supplied through the supply tube 3 into the reaction tube 1. The raw material gas comes into contact with the catalyst of the present invention in the reaction tube 1 to cause a reaction, and thus, 1,3-butadiene is generated. A generated gas 22 containing 1,3-butadiene is discharged through the discharge tube 4. The generated gas 22 may contain compounds such as acetaldehyde, propylene, and ethylene.

The generated gas 22 containing 1,3-butadiene is subjected, if necessary, to purification such as gas-liquid separation or distillation purification to remove an unreacted portion of the raw material and a by-product.

According to the present invention, 1,3-butadiene can be produced with the raw material conversion rate improved and with the selectivity of 1,3-butadiene increased. In addition, in the present invention, since the ethanol concentration in the raw material gas can be increased, the production amount per unit time of 1,3-butadiene can be increased, and hence production efficiency of 1,3-butadiene is high.

### Examples

The present invention will now be specifically described with reference to examples, and it is noted that the present invention is not limited to the following description.

### (Method for Measuring Amount of Bronsted Acid Sites)

After pyridine was adsorbed onto each of obtained catalysts by a method described below, an infrared absorption spectrum of the catalyst was observed by using an infrared spectrophotometer under measurement conditions of cumulative number of 100 times, resolution of 4, and absorbance measurement mode. The obtained spectrum was analyzed by using software, LabSolutions IR, manufactured by Shimadzu Corporation. A 3-point baseline correction function of the software was used to perform baseline correction based on signals at 2000 cm⁻¹, 1700 cm⁻¹ and 1400 cm⁻¹, and then, an automatic peak detection function of the software was used to perform peak detection with a threshold value set to 0.0001 and a noise level set to 0.0005. Thus, a corrected area of a highest peak in a range of 1540 to 1560 cm⁻¹ was calculated. Based on the thus obtained corrected area, an amount of Bronsted acid was calculated by using a molar extinction coefficient described in known literature (Journal of Catalysis, 1993, 141, 347-354). Results are shown in Table 1 below.

### [Measurement Method for Amount of Bronsted Acid]

Sample: 10 mg
Mold: disc mold having 20 Φ
Pretreatment: vacuum degassing (1.0 x 10⁻² Pa or less) at 500°C, followed by causing pyridine to pass therethrough at 150°C.
Name of apparatus: IRSpirit (manufactured by Shimadzu Corporation)

### Examples 1 to 9:

An impregnation solution prepared by dissolving hafnium (IV) chloride in ethanol was dropped onto a porous silica support (particle size: 1 to 4 µm, average pore diameter: 10.7 nm, total pore volume: 1.46 mL/g, specific surface area: 550 m²/g, manufactured by ACSMATERIAL), and the resultant was dried at 110°C for 3 hours, and fired at 400°C for 4.5 hours. An impregnation solution prepared by dissolving zinc nitrate hexahydrate in ethanol was dropped onto the porous silica support on which hafnium was supported, and the resultant was dried at 110°C for 3 hours, and fired at 400°C for 4.5 hours. Next, an impregnation solution prepared by dissolving alkali metal nitrate in water was dropped onto the porous silica support on which hafnium and zinc were supported, the resultant was dried at 80°C for 5 hours, and thus, a catalyst for butadiene synthesis was obtained. It is noted that use amounts of hafnium (IV) chloride, zinc nitrate hexahydrate, and alkali metal nitrate were adjusted so that content ratios of Hf, Zn and alkali metal in the catalytic metals could be those shown in Table 1. A prescribed amount of the catalyst for butadiene synthesis was weighed, subjected to a decomposition treatment by alkali fusion and acid dissolution, and adjusted in volume to be used as a test solution. Concentrations of Zn, Hf and the alkali metal in the test solution were measured using an ICP emission spectral analyzer.

### Comparative Examples 1 and 2:

Catalysts for 1,3-butadiene synthesis not containing an alkali metal were evaluated through procedures similar to those of Examples 1 to 9 except that the alkali metal nitrate was not used. Results are shown in Table 1.

An evaluation method for the catalysts for 1,3-butadiene synthesis prepared in Examples 1 to 9 and Comparative Examples 1 and 2 is as follows.

A reaction bed was formed by filling 3.4 g of each of the catalysts for 1,3-butadiene synthesis in a stainless steel cylindrical reaction tube having a diameter of 1/2 inches (1.27 cm) and a length of 15.7 inches (40 cm). Then, a reaction temperature (a temperature of the reaction bed) was set to 400°C or 350°C, a reaction pressure (a pressure in the reaction bed) was set to 0.1 MPa, and a raw material gas was supplied to the reaction tube at SV of 1200 L/hr/catalyst volume (L-catalyst) to obtain a generated gas. The raw material gas was a mixed gas of 30% by volume (in terms of a gas) of ethanol and 70% by volume (in terms of a gas) of nitrogen. The generated gas thus collected was analyzed by gas chromatography to obtain selectivity of 1,3-butadiene, acetaldehyde, ethylene and the rest of the compound, a raw material conversion rate, and a yield of 1,3-butadiene. The yield of 1,3-butadiene is a value calculated in accordance with [raw material conversion rate] x [selectivity of 1,3-butadiene]. These results are shown in Table 1.

### (1) Qualitative and Quantitative Analysis of Oxygen-containing Organic Compounds (Ethanol and Acetaldehyde)

Apparatus: SHIMADZU GC-2014, manufactured by Shimadzu Corporation
Column: Stabilwax, manufactured by Shimadzu GLC Ltd.
Analysis conditions: retained at 60°C for 4 min, heated at 10°C/min up to 180°C and retained at that temperature for 4 min, heated at 10°C/min up to 220°C, and retained at that temperature for 16 min

### (2) Qualitative and Quantitative Analysis of Hydrocarbon Compounds (1,3-Butadiene, Ethylene, and the like)

Apparatus: SHIMADZU GC-2014, manufactured by Shimadzu Corporation
Column: CP-Al2O3/KCl, manufactured by Agilent Technologies Japan, Ltd.
Analysis conditions: retained at 90°C for 20 min, and heated at 5°C/min up to 190°C

**[Table 1]**

| | Content Rate (mol%) of First Metal (Hf) | Content Rate (mol%) of Second Metal (Zn) | Third Metal | | Amount of Bronsted Acid Sites (µmol/g) | Reaction Temperature (°C) | Selectivity (%) | | | | Raw Material Conversion Rate (%) | Yield (%) of 1,3-butadiene |
|---|---|---|---|---|---|---|---|---|---|---|---|---|
| | | | Type | Content Rate (mol%) | | | 1,3-Butadiene | Acetaldehyde | Ethylene | Rest | | |
| Example 1 | 74.4 | 11.2 | Li | 14.4 | 1.6 | 400 | 43.7 | 5.8 | 7.5 | 43.0 | 98.7 | 43.2 |
| Example 2 | 74.4 | 11.2 | Na | 14.4 | 1.2 | 400 | 59.1 | 9.2 | 6.6 | 25.1 | 98.7 | 58.3 |
| Example 3 | 74.4 | 11.2 | K | 14.4 | 1.0 | 400 | 37.5 | 4.0 | 4.7 | 53.8 | 98.7 | 37.0 |
| Example 4 | 74.4 | 11.2 | Cs | 14.4 | 1.1 | 400 | 49.8 | 9.0 | 4.5 | 36.7 | 97.7 | 48.4 |
| Example 5 | 76.6 | 11.5 | Na | 11.9 | 1.8 | 400 | 45.3 | 3.2 | 8.6 | 42.9 | 99.1 | 44.9 |
| Example 6 | 72.3 | 10.9 | Na | 16.8 | 1.6 | 400 | 53.2 | 10.7 | 6.3 | 29.8 | 97.1 | 51.7 |
| Example 7 | 68.5 | 10.3 | Na | 21.2 | 0.3 | 400 | 49.1 | 10.5 | 5.0 | 35.4 | 97.4 | 47.8 |
| Example 8 | 65 | 9.8 | Na | 25.2 | 0.0 | 400 | 40.4 | 20.6 | 2.4 | 36.6 | 92.3 | 37.3 |
| Example 9 | 74.4 | 11.2 | Na | 14.4 | 1.2 | 350 | 63.0 | 18.0 | 7.0 | 12.0 | 63.0 | 40.0 |
| Comparative Example 1 | 86.9 | 13.1 | - | - | 1.9 | 400 | 35.9 | 1.9 | 10.4 | 51.8 | 99.5 | 35.7 |
| Comparative Example 2 | 86.9 | 13.1 | - | - | 1.9 | 350 | 58.5 | 10.0 | 25.0 | 6.5 | 60.7 | 35.5 |

When 1,3-butadiene was synthesized from the raw material gas containing ethanol at a high concentration of 30% by volume, the raw material conversion rates of the catalysts for butadiene synthesis of Examples 1 to 9 and the selectivity of 1,3-butadiene to be synthesized were high, resulting in a high yield of 1,3-butadiene. In contrast, in Comparative Examples 1 and 2 not containing the alkali metal, either the raw material conversion rate of the catalyst for 1,3-butadiene synthesis or the selectivity of 1,3-butadiene to be synthesized was low, resulting in a low yield of 1,3-butadiene.

### Reference Signs List

1 ... reaction tube, 2 ... reaction bed, 3 ... supply tube, 4 ... discharge tube, 5 ... temperature controller, 6 ... pressure controller, 10 ... butadiene production apparatus

## Claims

1. A catalyst comprising at least one first metal selected from the group consisting of Groups 3 to 6 of the periodic table,
wherein the catalyst has an amount of Bronsted acid sites of 1.8 µmol/g or less.

2. A catalyst comprising:
at least one first metal selected from the group consisting of Groups 3 to 6 of the periodic table;
at least one second metal selected from the group consisting of Groups 12 to 14 of the periodic table; and
an ethylene suppressant.

3. The catalyst according to claim 2, wherein the ethylene suppressant is at least one third metal selected from the group consisting of Groups 1 and 2 of the periodic table.

4. The catalyst according to any one of claims 1 to 3, wherein the first metal is hafnium.

5. The catalyst according to claim 3 or 4, wherein the third metal comprises at least one selected from the group consisting of Li, Na, K, Rb, Cs, Mg, Ca, Sr, and Ba.

6. The catalyst according to any one of claims 3 to 5, wherein a content rate of the third metal to a total number of moles of the first metal, the second metal and the third metal is 5 to 30% by mole.

7. The catalyst according to any one of claims 1 to 6, for synthesizing 1,3-butadiene from ethanol.

8. The catalyst according to any one of claims 3 to 7, comprising a support on which the first metal, the second metal and the third metal are supported.

9. A method for producing 1,3-butadiene, comprising contacting a raw material gas comprising ethanol with the catalyst according to any one of claims 1 to 8.

10. The method for producing 1,3-butadiene according to claim 9, wherein a content rate of the ethanol in the raw material gas is 10% by volume or more with respect to 100% by volume of the raw material gas.

11. The method for producing 1,3-butadiene according to claim 9 or 10, wherein a reaction for the contacting is performed at 200 to 800°C.
